# EUROPÄISCHE PATENTSCHRIFT

(11) **EP 2 703 034 B1**
(45) Veröffentlichungstag und Bekanntmachung des Hinweises auf die Patenterteilung: **17.05.2017**
(21) Anmeldenummer: 13004229.4
(22) Anmeldetag: 27.08.2013
(51) Int. Cl.: A61M 16/16, A61M 16/10

(54) **Vorrichtung zur Anfeuchtung von Atemgas**
Device for the humidification of respiratory gas
Dispositif d'humidification de gaz respiratoire

(30) Priorität: 28.08.2012 DE 102012016972
(43) Veröffentlichungstag der Anmeldung: 05.03.2014
(73) Patentinhaber: Löwenstein Medical Technology S.A., 2557 Luxembourg (LU)
(72) Erfinder: Feldhahn, Karl-Andres, 22761 Hamburg (DE); Hein, Stefan, 22529 Hamburg (DE); König, Karl-Heinz, 22850 Norderstedt (DE); Sepke, Heiko, 22952 Lütjensee (DE); Gardein, Joachim, 38430 Icod de los Vinos, Tenerife (ES)
(74) Vertreter: Marx, Thomas

(56) Entgegenhaltungen:
- WO-A1-02/066106
- DE-U1-202004 021 774
- US-A1- 2006 237 005
- US-A1- 2008 072 900
- US-A1- 2008 257 346
- US-A1- 2011 132 360
- US-A1- 2011 155 132
- US-B1- 6 435 180

## Beschreibung

Die Erfindung betrifft eine Vorrichtung zur Anfeuchtung von Atemgas.

Eine typische Verwendung von Atemluftanfeuchtern erfolgt in Zusammenhang mit Atemluftversorgungen, die im Rahmen einer CPAP-Therapie (Continious-Positive-Airway-Pressure), Bilevel-, APAP- oder Heimbeatmung eingesetzt werden. Sie vermeiden ein Austrocknen der Atemwege insbesondere bei längeren Beatmungsphasen. Der Atemluftanfeuchter weist in der Regel einen befüllbaren Wassertank und ein Heizelement auf.

Aufgrund der einzuhaltenden hygienischen Anforderungen an einen Atemluftanfeuchter, muss dieser leicht zu demontieren bzw. zu montieren und zu reinigen sein.

Bei der DE10049869A1 wird Atemluftanfeuchter zwischen Patientenschlauchsystem und Beatmungsgerät angeordnet. Dadurch muss zur Reinigung und auch zur Befüllung des Anfeuchters das Patientenschlauchsystem demontiert werden, was einen erhöhten Aufwand für die Pflege und Reinigung bedeutet. Auch bei der DE102006034028A1 muss zur Reinigung und auch zur Befüllung des Anfeuchters das Patientenschlauchsystem demontiert werden.

Die US2011155132 A1 offenbart einen Atemluftanfeuchter zur Verwendung mit einem Beatmungsgerät bestehend aus einem Gehäuseunterteil mit einem passiven Heizelement einem Gehäuseoberteil und einem elastomeren Mittelteil wobei das Mittelteil eine Dichtauflage zum Gehäuseunterteil aufweist. Der Anfeuchter wird neben dem Beatmungsgerät platziert und dort über den Schlauchanschluss des Beatmungsgerätes konnektiert. Der Luftweg geht dann durch den Anfeuchter zum Schlauchanschluss des Anfeuchters und weiter zum dort befestigten Patientenschlauch.

Die US2006237005 A1 offenbart einen Atemluftanfeuchter zur Verwendung mit einem Beatmungsgerät bestehend aus einem Gehäuseunterteil mit einen passiven Heizelement einem Gehäuseoberteil und einem elastomeren Mittelteil wobei das Mittelteil eine Dichtauflage zum Gehäuseunterteil aufweist. Der Anfeuchter wird vor dem Beatmungsgerät platziert und dort über den Schlauchanschluss des Beatmungsgerätes konnektiert. Der Luftweg geht dann durch den Anfeuchter zum Schlauchanschluss des Anfeuchters und weiter zum dort befestigten Patientenschlauch.

Die DE202004021774 U1, die US2008072900 A1 und die WO02066106 A1 offenbaren jeweils einen Atemluftanfeuchter zur Verwendung mit einem Beatmungsgerät der ähnlich baut wie der der US2006237005 A1.

US2008257346 A1 offenbart einen mehrteiligen Schalldämpfer mit Atemluftanfeuchter zur Verwendung mit einem Beatmungsgerät.

Die US6435180 B1 offenbart einen Atemluftanfeuchter zur Verwendung mit einem Beatmungsgerät bestehend aus einem Gehäuseunterteil mit einen passiven Heizelement einem Gehäuseoberteil und einem elastomeren Mittelteil wobei das Mittelteil eine Dichtauflage zum Gehäuseunterteil aufweist. Der Anfeuchter wird über eine Schlauchverbindung mit dem Beatmungsgerät konnektiert. Nachteilig ist hierbei, dass keine konstruktive Verbindung zum Beatmungsgerät besteht.

Die US 2011/132360 A1 offenbart einen Atemluftanfeuchter zur Verwendung mit einem Beatmungsgerät bestehend aus einem

Gehäuseunterteil mit Heizelement einem Gehäuseoberteil und einem elastomeren Mittelteil wobei das Mittelteil eine Dichtauflage zum Gehäuseunterteil darstellt. Der Anfeuchter wird auf dem Beatmungsgerät platziert und dort mit dem Schlauchanschluss konnektiert. Der Luftweg verläuft im Bypass durch den Anfeuchter und wieder zurück in das Beatmungsgerät, wobei der Schlauchanschluss lösbar ist und auch dazu dient, den Anfeuchter zu verrasten.

Nachteilig ist hierbei, sowie bei allen weiteren Lösungen aus dem Stand der Technik, dass der Schlauch und/oder der Schlauchanschluss vom Beatmungsgerät entfernt werden muss, um den Anfeuchter zu konnektieren.

Nachteilig bei der US 2011/132360 A1 ist außerdem, dass der Anfeuchter Elemente zur Strömungsführung und/oder Strömungskanäle im Oberteil und im Unterteil aufweist. Die Herstellung von komplexen Elementen zur Strömungsführung und/oder Strömungskanälen durch werkzeuggebundene Spritzgussverfahren im Hartkunststoff ist wesentlich schwieriger als bei elastomeren Teilen, weshalb konstruktive Einschränkungen bestehen. Zudem muss in einem zweiten Schritt das lediglich als Dichtung dienende Mittelteil eingesetzt werden.

Alle bekannten Anfeuchter sind vielteilig aufgebaut. Die große Anzahl an trennbaren Einzelbestandteilen erschwert die Montage und Demontage und somit Reinigung der Anfeuchter.

Ziel der Erfindung ist es einen Atemluftanfeuchter bereitzustellen, der an die Bedürfnisse des Anwenders angepasst ist.

Erfindungsgemäß wird das Ziel erreicht durch einen Atemluftanfeuchter nach Anspruch 1 zur Verwendung mit einem Beatmungsgerät bestehend aus einem Gehäuseunterteil mit Heizelement, einem Gehäuseoberteil und einem elastomeren Mittelteil wobei das Mittelteil eine Dichtauflage zum Gehäuseunterteil aufweist und wobei der Anfeuchter in einem Seitenbereich an das Beatmungsgerät konnektiert wird und der Luftweg im Bypass durch den Anfeuchter und wieder zurück in das Beatmungsgerät geführt wird, wobei der Schlauchanschluss, an den ein Beatmungsschlauch mit Beatmungsmaske oder Beatmungsbrille angeschlossen werden kann, am Beatmungsgerät verbleibt und wobei das Mittelteil einstückig ausgebildet ist und Elemente zur Strömungsführung und/oder Strömungskanäle und Dichtelemente zur pneumatischen Abdichtung aufweist.

In dem Ausführungsbeispiel ist der Atemluftanfeuchter als modulares Funktionselement ausgeführt, welches bei Bedarf mit nur wenigen Handgriffen an das Beatmungsgerät konnektiert und so den individuellen Bedürfnissen des Anwenders angepasst wird.

Der Atemluftanfeuchter ist zur Verwendung mit einem Beatmungsgerät vorgesehen und besteht aus einer gegenüber dem Stand der Technik reduzierten Anzahl an Teilen, nämlich einem Gehäuseunterteil mit einem direkten oder indirekten Heizelement, einem Gehäuseoberteil und einem elastomeren Mittelteil, welches einstückig ist und eine Dichtauflage, die zumindest teilweise als Dichtfläche ausgestaltet sein kann, zum Gehäuseunterteil aufweist.

Der erfindungsgemäße Anfeuchter besteht somit bevorzugt aus sechs , fünf, oder vier manuell trennbaren Einzelbauteilen.

Dies wird insbesondere erreicht durch eine Funktionsintegration im Bereich des Mittelteiles. Das Mittelteil ist bevorzugt einstückig ausgebildet und dient der Strömungsführung, der Schalldämmung, der Befüllung mit Wasser und der Wasserführung, der Abdichtung zwischen Ober- und Unterteil, sowie der Abdichtung zum Beatmungsgerät hin.

Der Atemluftanfeuchter ist bevorzugt so aufgebaut, dass das Mittelteil einstückig ausgebildet ist und Elemente zur Strömungsführung und/oder Strömungskanäle und Dichtelemente zur pneumatischen Abdichtung aufweist.

Der Atemluftanfeuchter ist zur Verwendung mit einem Beatmungsgerät vorgesehen und besteht aus einem Gehäuseunterteil mit direkten oder indirekten Heizelement, einem Gehäuseoberteil und einem elastomeren Mittelteil, welches eine Dichtauflage, die zumindest teilweise als Dichtfläche ausgestaltet sein kann, zum Gehäuseunterteil aufweist.

Der Atemluftanfeuchter weist eine kompakte Gehäusekonstruktion auf, die dem Konturverlauf des Beatmungsgerätes angepasst ist. Der Atemluftanfeuchter ist auch dadurch gekennzeichnet, dass im Gehäuseoberteil als Öffnungen ein Lufteinlass und ein Luftauslass zur luftführenden Kopplung mit dem Beatmungsgerät vorgesehen sind.

Der Atemluftanfeuchter ist auch dadurch gekennzeichnet, dass der Lufteinlass und/oder der Luftauslass auch als Einfüllöffnungen für das Wasser dienen.

Der Atemluftanfeuchter ist auch dadurch gekennzeichnet, dass räumlich benachbart zu der Öffnung zumindest eine erhöhte Kante als Überlaufschutz angeordnet ist.

Der Atemluftanfeuchter ist auch dadurch gekennzeichnet, dass die Fläche im Bereich neben der Öffnung leicht trichterförmig ausgeführt ist, um das einzufüllende Wasser in Richtung der Öffnung zu führen.

Der Atemluftanfeuchter ist auch dadurch gekennzeichnet, dass im Gehäuseoberteil zumindest ein Griff bereitgestellt wird.

Der Atemluftanfeuchter ist auch dadurch gekennzeichnet, dass im Gehäuseunterteil zwei von außen sichtbare Füllstandanzeigen angeordnet sind, die eine maximale Füllhöhe anzeigen.

Der Atemluftanfeuchter ist auch dadurch gekennzeichnet, dass durch die gekrümmt angeordneten Luftkanäle des Mittelteiles zudem ein Zurück- bzw. Auslaufen der Flüssigkeit aus dem Anfeuchter verhindern wird.

Der Atemluftanfeuchter ist auch dadurch gekennzeichnet, dass eine einhändige Konnektierung des Anfeuchters an das Beatmungsgerät durch korrespondierende Abmessungen im Bereich der Kontaktstelle und einen Schnappmechanismus zur Befestigung erreicht wird.

Der Atemluftanfeuchter ist auch dadurch gekennzeichnet, dass der Anfeuchter in einem Seitenbereich an das Beatmungsgerät konnektiert wird und der Luftweg im Bypass durch den Anfeuchter und wieder zurück in das Beatmungsgerät geführt wird, wobei der Schlauchanschluss am Beatmungsgerät verbleibt.

Der Atemluftanfeuchter ist auch dadurch gekennzeichnet, dass das Mittelteil zumindest ein Dichtelement zur Abdichtung zum Beatmungsgerät hin aufweist.

Der Atemluftanfeuchter ist auch dadurch gekennzeichnet, dass das Mittelteil aus mindestens 2 Elastomeren verschiedener Härtegrade (Shorehärten) besteht.

Der Atemluftanfeuchter ist auch dadurch gekennzeichnet, dass das elastomere Mittelteil in einer 2K- Technik hergestellt wird.

Der Atemluftanfeuchter ist auch dadurch gekennzeichnet, dass die strömungsführenden Elemente des elastomeren Mittelteils eine höhere Shorehärte und die Dicht(-auflage)-elemente des elastomeren Mittelteils eine niedrigere Shorehärte aufweisen.

Der Atemluftanfeuchter ist auch dadurch gekennzeichnet, dass die strömungsführenden Elemente des elastomeren Mittelteils eine Shorehörte zwischen 40 und 100 Shore A und die Dicht(-auflage) -elemente des elastomeren Mittelteils eine Shorehärte zwischen 5 und 70 Shore A aufweisen.

Der Atemluftanfeuchter ist auch dadurch gekennzeichnet, dass die Dicht(-auflage)-elemente des elastomeren Mittelteils den von Mittelteil und Gehäuseunterteil gemeinsam gebildeten Wasseraufnahmeraum gegenüber der Umgebung abdichten.

Der Atemluftanfeuchter ist auch dadurch gekennzeichnet, dass das Mittelteil Elemente zur Strömungsführung und/oder Strömungskanäle aufweist.

Der Atemluftanfeuchter ist auch dadurch gekennzeichnet, dass die Elemente zur Strömungsführung und/oder Strömungskanäle mindestens eine Umlenkung der Luft von horizontal nach vertikal und/oder eine Umlenkung von vertikal in eine horizontale Strömungsrichtung zum Anfeuchterausgang hin bewirken.

Der Atemluftanfeuchter ist auch dadurch gekennzeichnet, dass die Strömungsführungselemente des Mittelteils mindestens eine Umlenkung aus einer horizontalen Strömungsrichtung (vom Beatmungsgerät kommend) in eine vertikale Strömungsrichtung (auf das Wasser zu) bewirken.

Der Atemluftanfeuchter ist auch dadurch gekennzeichnet, dass die Strömungsführungselemente des elastomeren Mittelteils mindestens eine Umlenkung aus einer vertikalen Strömungsrichtung (vom Wasser weg) in eine horizontale Strömungsrichtung (zum Beatmungsgerät bzw. zum Geräteausgang hin) bewirken.

Der Atemluftanfeuchter ist auch dadurch gekennzeichnet, dass die Strömungsführungselemte des Mittelteils horizontale Umlenkungen in der Luftströmung oberhalb der Wasseroberfläche bewirken und durch diese Umlenkungen bei gegebenem Anfeuchterraum einen langen Strömungsweg der Luft über dem Wasser zur Aufnahme von Wassermolekülen in der Luft erzwingen.

Der Atemluftanfeuchter ist auch dadurch gekennzeichnet, dass die Elemente zur Strömungsführung und/oder Strömungskanäle eine Aufteilung der Luftströmung in mindestens zwei Teilströme oberhalb der Wasseroberfläche bewirken und die Teilströme vor dem Luftauslass wieder vereinigt werden.

Der Atemluftanfeuchter ist auch dadurch gekennzeichnet, dass das Mittelteil des Atemluftanfeuchters zum Einen der Luftführung durch den Anfeuchter, zum Anderen der Abdichtung zwischen Ober- und unterteil und auch der Wasserführung beim Befüllvorgang dient.

Der Atemluftanfeuchter ist auch dadurch gekennzeichnet, dass der Lufteinlass und der Luftauslass auch als Einfüllöffnungen für das Wasser dienen.

Der Atemluftanfeuchter ist auch dadurch gekennzeichnet, dass im Gehäuseoberteil als Öffnungen ein Lufteinlass und ein Luftauslass zur luftführenden Kopplung mit dem Beatmungsgerät vorgesehen sind.

Der Atemluftanfeuchter ist auch dadurch gekennzeichnet, dass das elastomere Mittelteil rohrförmige Anschlussstellen bereitstellt die mit den Öffnungen im Gehäuseoberteil gemeinsam jeweils einen Lufteinlass und einen Luftauslass bilden.

Der Atemluftanfeuchter ist auch dadurch gekennzeichnet, dass die rohrförmigen Anschlussstellen im elastomeren Mittelteil im Kopplungsbereich zum Beatmungsgerät Dichtelemente zur Abdichtung zum Beatmungsgerät hin aufweisen.

Der Atemluftanfeuchter ist auch dadurch gekennzeichnet, dass die Dichtelemente an den rohrförmigen Anschlussstellen eine Shorehärte zwischen 5 und 70 Shore A aufweisen.

Der Atemluftanfeuchter ist auch dadurch gekennzeichnet, dass Lufteinlass und Luftauslass benachbart angeordnet sind und eine Befüllung mit Wasser wahlweise durch eine der beiden Öffnungen vorgenommen werden kann.

Der Atemluftanfeuchter ist auch dadurch gekennzeichnet, dass Luftkanäle im Mittelteil beim Übergang zwischen vertikalen und horizontalen Abschnitten geometrische Überhöhungen enthalten, um während des Betriebes oder bei Transport und Verkippen ein Eindringen von Wasser in die horizontalen Strömungsabschnitte (zurück in das Gerät oder hin zum Geräteausgang) zumindest zu erschweren.

Der Atemluftanfeuchter ist auch dadurch gekennzeichnet, dass die vertikalen Luftkanäle im Bereich der Mitte des Anfeuchters angeordnet sind um während des Betriebes oder bei Transport und bei Verkippen ein Eindringen von Wasser in die horizontalen Strömungsabschnitte (zurück in das Gerät oder hin zum Geräteausgang) zumindest zu erschweren.

Der Atemluftanfeuchter ist auch dadurch gekennzeichnet, dass das Gehäuseoberteil im Wesentlichen der mechanischen Abstützung des elastomeren Mittelteils sowie der mechanischen Verriegelung mit dem Unterteil dient, wobei das Mittelteil zwischen Oberteil und Unterteil eingespannt wird.

Der Atemluftanfeuchter ist auch dadurch gekennzeichnet, dass zum Zusammenbau zunächst in einem einfachen Montageschritt das elastomere Mittelteil in das Oberteil eingesetzt wird woraufhin in einem zweiten ebenfalls einfachen Montageschritt die Einheit aus Gehäuseoberteil und elastomeren Mittelteil mit dem Gehäuseunterteil einrastend zusammengesetzt wird.

Der Atemluftanfeuchter ist auch dadurch gekennzeichnet, dass das Einrasten durch Rasthaken im Unterteil und entsprechende Öffnungen für die Rasthaken im Oberteil vorgesehen sind.

Der Atemluftanfeuchter ist auch dadurch gekennzeichnet, dass im Gehäuseunterteil Führungsaufnahmen zur Justierung des Gehäuseoberteiles angeordnet sind, in die entsprechende Führungsstege des Gehäuseoberteiles greifen die einen falschen Zusammenbau der Bauteile verhindern.

Der Atemluftanfeuchter ist auch dadurch gekennzeichnet, dass die Führungsstege und -aufnahmen asymmetrisch an Gehäuseunterteil und Oberteil angeordnet sind um eine definierte Orientierung bei der Montage vorzugeben.

Der Atemluftanfeuchter ist auch dadurch gekennzeichnet, dass die Führungsaufnahmen jeweils eine trichterförmige Öffnung aufweisen die zum Einführen der Führungsstege in die Führungsaufnahmen dienen.

Der Atemluftanfeuchter ist auch dadurch gekennzeichnet, dass zum Lösen der Verbindung zwischen Oberteil und Unterteil eine Grifffläche im Bereich unter einen Schenkel des Oberteiles vorgesehen ist und dass durch eine leichte Zugbewegung die Rastverbindung gelöst werden kann.

Der Atemluftanfeuchter ist auch dadurch gekennzeichnet, dass im Gehäuseoberteil zumindest ein Griff bereitgestellt wird.

Der Atemluftanfeuchter ist auch dadurch gekennzeichnet, dass im Gehäuseunterteil zwei von außen sichtbare Füllstandanzeigen angeordnet sind, die eine Füllhöhe anzeigen.

Der Atemluftanfeuchter ist auch dadurch gekennzeichnet, dass im Gehäuseunterteil mindestens zwei von außen sichtbaren Füllstandanzeigen zum Ablesen bei verschiedenen räumlichen Winkellagen angeordnet sind, die eine maximale Füllhöhe anzeigen.

Der Atemluftanfeuchter ist auch dadurch gekennzeichnet, dass eine einhändige Konnektierung des Anfeuchters an das Beatmungsgerät durch korrespondierende Abmessungen im Bereich der Kontaktstelle und einen Schnappmechanismus zur Befestigung erreicht wird.

Der Atemluftanfeuchter ist auch dadurch gekennzeichnet, dass der Anfeuchter in einem Seitenbereich an das Beatmungsgerät konnektiert wird und der Luftweg durch den Anfeuchter und wieder zurück in das Beatmungsgerät geführt wird, wobei der Schlauchanschluss am Beatmungsgerät verbleibt.

Mittelteil für einen Atemluftanfeuchter ist dadurch gekennzeichnet, dass das Mitteilteil Elemente zur Strömungsführung und/oder Strömungskanäle aufweist, die aufgrund ihrer räumlichen Gestalt bei einem werkzeugbasierten Herstellungsprozesses elastische Nachgiebigkeit des Mittelteiles bei der Entformung voraussetzen.

Werkzeuggebundenes Verfahren zur Herstellung eines elastomeren Mittelteils für einen Atemluftanfeuchter dadurch gekennzeichnet, dass in dem Mittelteil zumindest ein in Richtung der Öffnung des Werkzeugs realisierter Hinterschnitt unter Beanspruchung der Nachgiebigkeit des elastomeren Werkstoffs mindestens bereichsweise zwangsentformt wird.

Werkzeuggebundenes Verfahren zur Herstellung eines elastomeren Mittelteils für einen Atemluftanfeuchters dadurch gekennzeichnet, dass das Mittelteil strömungsführende Elemente zur Realisierung von Umlenkungen der Strömung innerhalb des Atemluftanfeuchters bewirken wobei in dem Mittelteil in Richtung der Öffnung des Werkzeugs realisierten Hinterschnitte (Entformung) unter Beanspruchung der Nachgiebigkeit des elastomeren Werkstoffs mindestens bereichsweise zwangsentformt werden.

Im Gehäuseoberteil sind als Öffnungen ein Lufteinlass und ein Luftauslass zur luftführenden Kopplung mit dem Beatmungsgerät vorgesehen.

Der Anfeuchter wird im Seitenbereich des Beatmungsgerätes angekoppelt, wobei die luftführende Verbindung in Form eines Bypass erfolgt.

Der Lufteinlass und der Luftauslass - die Öffnungen im Oberteil - dienen auch als Einfüllöffnungen für das Wasser.

Im Gehäuseoberteil wird zumindest ein Griff bereitgestellt. Ein Griff dient dazu, das Oberteil vom Unterteil zu lösen.

Im Gehäuseunterteil sind zwei von außen sichtbare Füllstandanzeigen angeordnet, die eine maximale Füllhöhe anzeigen. Eine Füllstandanzeige befindet sich im Bodenbereich des Gehäuseunterteils. Das Gehäuseunterteil ist dazu zumindest teilweise aus einem transparenten Material hergestellt.

Bei Befüllung des Anfeuchters über die Öffnung fließt das Wasser, den vom Mittelteil gebildeten Kanal entlang, in das Unterteil. Dort steigt der Wasserstand im Wasseraufnahmeraum bis zum oberen Rand des Kanals an, dann kann die Luft im Anfeuchter nicht mehr durch den Kanal entweichen und mit weiter steigendem Wasserstand wird die Luft komprimiert wobei der Druck F_{L} der im Luftraum eingeschlossenen Luft auf das im Wasseraufnahmeraum des Anfeuchters befindliche Wasser ansteigt; so entspricht dann der Wasserstand der maximalen Füllhöhe und bei weiterer Befüllung über die Öffnung fließt kein weiteres Wasser in den Wasseraufnahmeraum.

Die gekrümmt angeordneten Luftkanäle des Mittelteiles verhindern zudem ein Zurück- bzw. Auslaufen der Flüssigkeit aus dem Anfeuchter. Die zentrierte Anordnung der Kanäle trägt ebenfalls dazu bei.

Das Mittelteil des Atemluftanfeuchters dient zum Einen der Luftführung durch den Anfeuchter, zum Anderen der Abdichtung zwischen Ober- und Unterteil und auch der Wasserführung beim Befüllvorgang.

Eine einhändige Konnektierung des Anfeuchters an das Beatmungsgerät wird durch korrespondierende Abmessungen im Bereich der Kontaktstelle und einen Schnappmechanismus zur Befestigung erreicht.

Der Anfeuchter wird in einem Seitenbereich an das Beatmungsgerät konnektiert und der Luftweg wird im Bypass durch den Anfeuchter und wieder zurück in das Beatmungsgerät geführt, wobei der Schlauchanschluss am Beatmungsgerät verbleibt.

In den folgenden Zeichnungen sind Ausführungsbeispiele dargestellt. Es zeigen:
- Fig. 1: Aufbau einer Atemgasversorgung ohne Anfeuchter
- Fig. 2: Beatmungsgerät mit abgenommenem Seitenteil
- Fig. 3: Anfeuchter mit seitlicher Aufnahme des Beatmungsgerätes
- Fig. 4: Aufbau eines Beatmungsgerätes mit angeschlossenem. Anfeuchter
- Fig. 5: Anfeuchter
- Fig. 6: Anfeuchter zum Befüllen geschwenkt
- Fig. 7: Explosionszeichnung des Anfeuchters
- Fig. 8: Perspektive Gehäuseunterteil
- Fig. 9: Unteransicht Gehäuseunterteil
- Fig.10: Mittelteil
- Fig.10a: Schnitt durch Mittelteil
- Fig.11: Gehäuseoberteil
- Fig.12: Schema Anfeuchter, Befüllvorgang

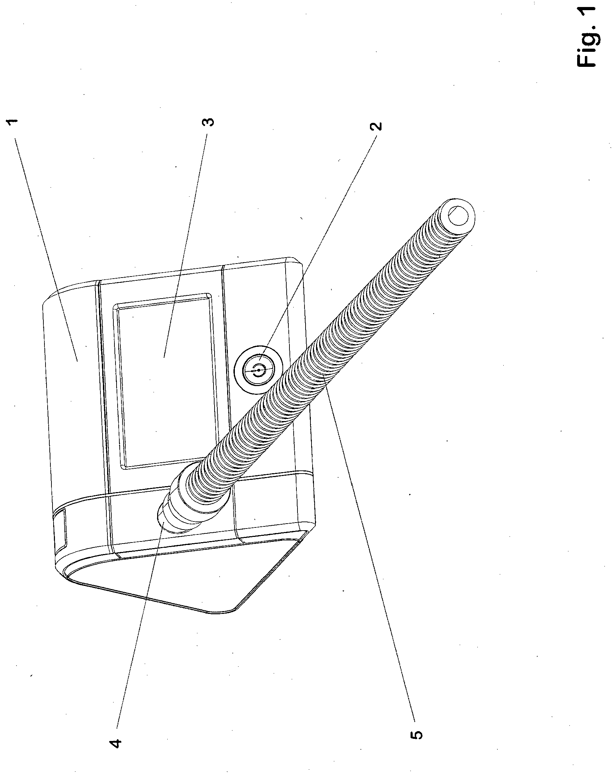

Fig. 1 zeigt den grundsätzlichen Aufbau einer Atemgasversorgung, welche ohne Atemluftanfeuchter ausgeführt ist. Die Atemgasversorgung (1) weist ein Bedienfeld (2) sowie eine Anzeige mit Touchscreen (3) auf. Die Atemgasversorgung ist darüber hinaus im Geräteinnenraum mit einer Fördereinrichtung für das Atemgas in Form einer Atemgaspumpe, einem elektrischen Antrieb, sowie einer Steuerung für den elektrischen Antrieb ausgestattet. Über ein Anschlusselement (4) wird ein Beatmungsschlauch (5) angeschlossen an dessen Ende sich die Atemgasversorgung des Patienten in Form einer Beatmungsmaske oder Beatmungsbrille befindet.

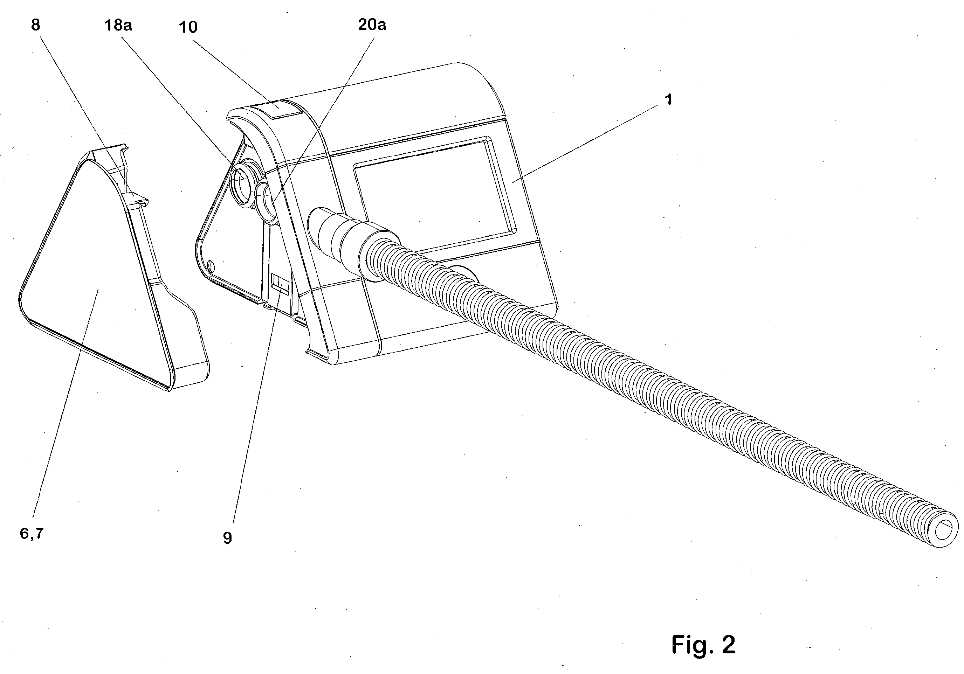

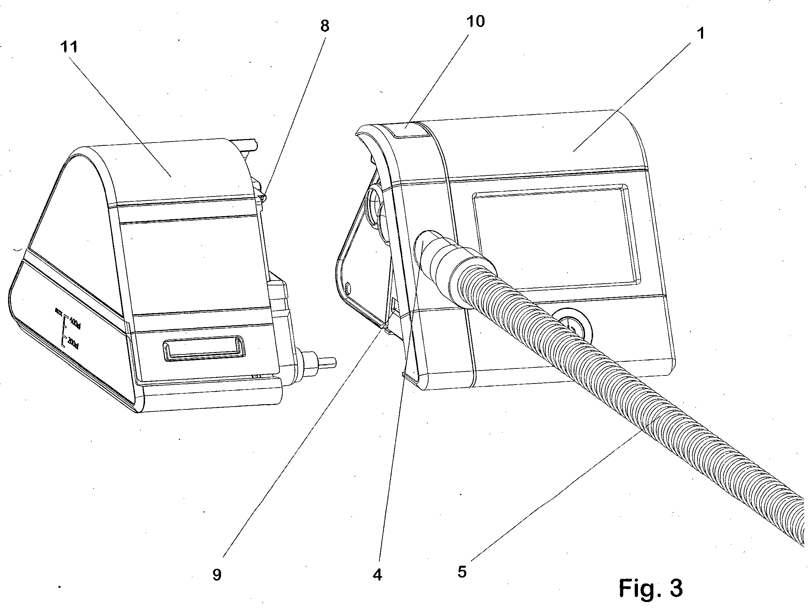

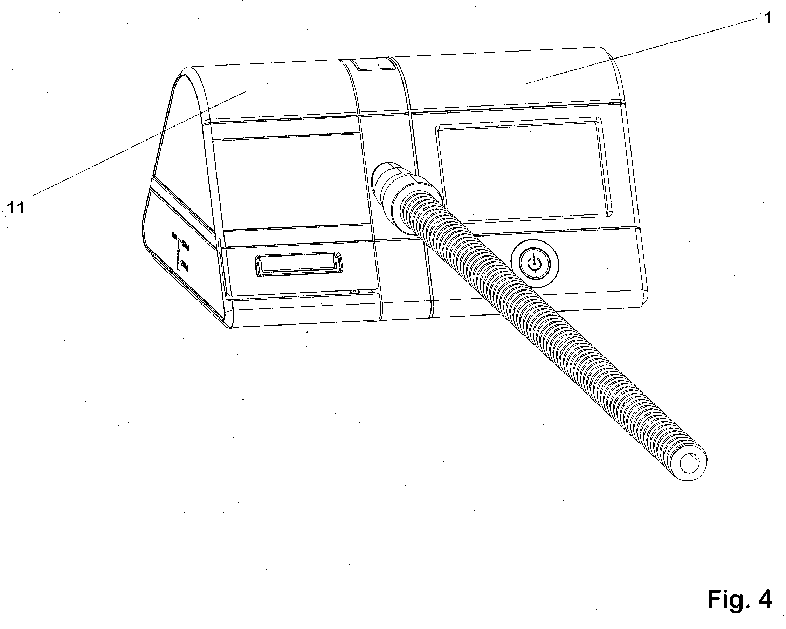

Fig.2: Um den Atemluftanfeuchter (11) an das Beatmungsgerät (1) zu koppeln wird die Seitenwand (6) des Beatmungsgerätes (1), die einen integrierten Schalldämpfer (7) aufweist, der mit zwei Schnapphaken (8) in Aufnahmen (9) im Beatmungsgerät (1) verrastet, gelöst und gegen das Atemluftanfeuchtermodul (11) ausgetauscht. Das Beatmungsgerät weist im Seitenbereich eine Luftaustritts- (18a) und eine Lufteintrittsöffnung (20a) auf, die entweder im Bypass über den Schalldämpfer verbunden werden oder über den Anfeuchter (11). Hierzu muss die Entriegelung (10) am Beatmungsgerät (1) betätigt werden. Durch Betätigen der Entriegelung (10) werden die Aufnahmen (9), die auf einer Verbindungsschiene miteinander verbunden sind, verschoben und die Verbindung zu den Schnapphaken (8) gelöst. Der Atemluftanfeuchter (11) weist ebenfalls je einen Schnapphaken (8) im Oberteil und Unterteil auf, die in die gleichen Aufnahmen (9) des Beatmungsgerätes (1) greifen. Dieses wird in den Figuren 2 bis 4 dargestellt.

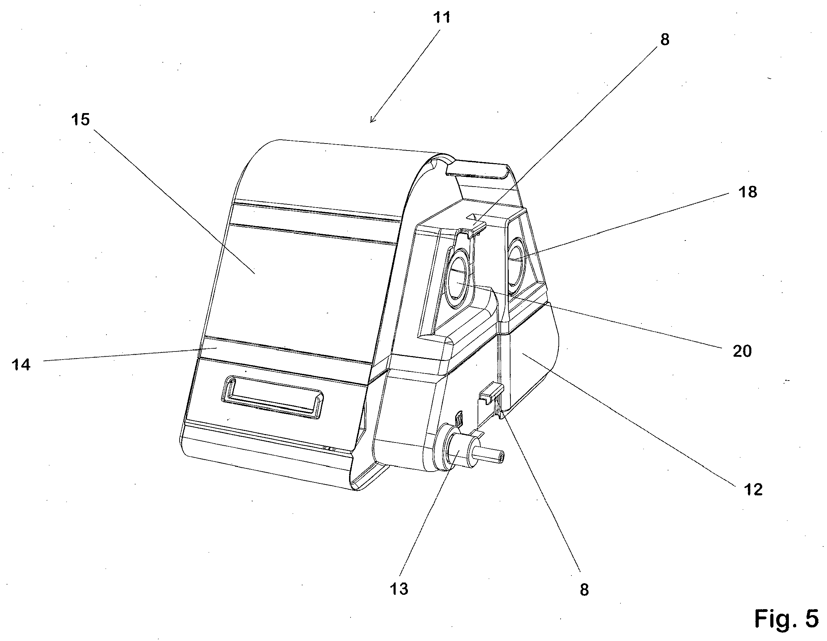

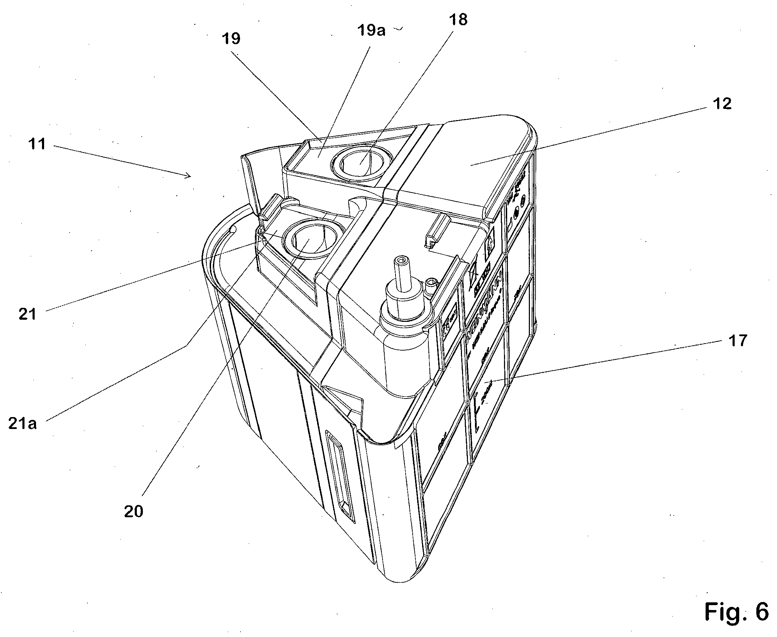

Die Figuren 5 und 6 zeigen den Atemluftanfeuchter (11) im montierten Zustand, welcher aus einem Gehäuseunterteil (12) mit Heizelement (13), einem Mittelteil bevorzugt aus Silikon (14) mit einer Dichtauflage (22a) zum Gehäuseunterteil (12) und einem Gehäuseoberteil (15) besteht.

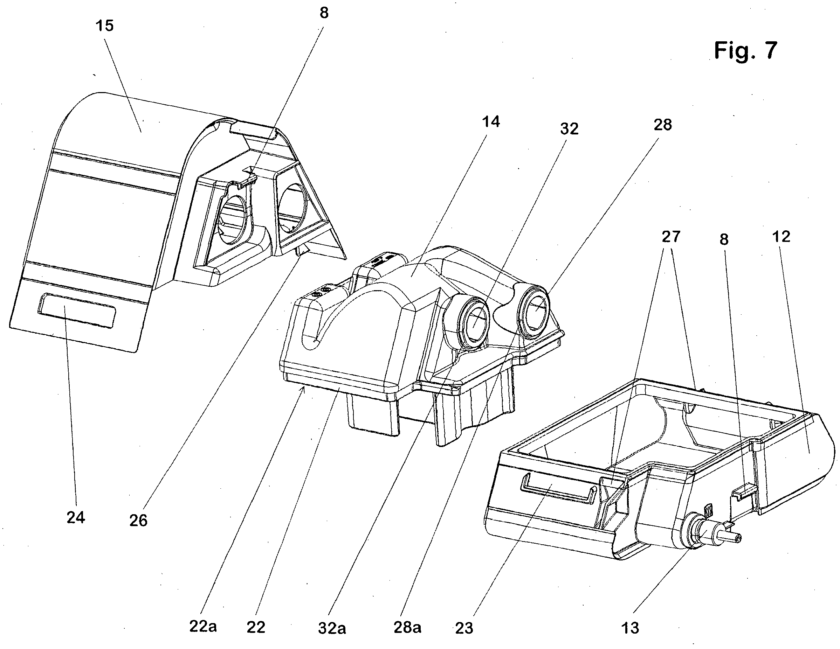

Fig. 7 zeigt in einer Explosionsansicht die Einzelteile des Anfeuchtermoduls (11). Zur Montage wird das Mittelteil aus Elastomer, bevorzugt Silikon (14), in das Gehäuseoberteil (15) eingesetzt. Das Mittelteil dichtet mit der Dichtfläche (22a) seines Dichtrandes (22) auf dem Unterteil (12) ab, wenn Ober-(15) und Unterteil (12) miteinander verbunden werden. Das Verbinden der Teile erfolgt über Rasthaken (23) am Unterteil (12) und entsprechenden Öffnungen (24) für die Rasthaken (23) im Oberteil. Zum Lösen der Verbindung ist eine Grifffläche (25) im Bereich unter dem Schenkel des Oberteiles (15) vorgesehen. Eine korrespondierende Aussparung (25a) im Unterteil ermöglicht das Greifen. Mit leichter Zugbewegung lösen sich die Rastverbindungen (23,24) aufgrund der Elastizität des verwendeten KunststoffMaterials.

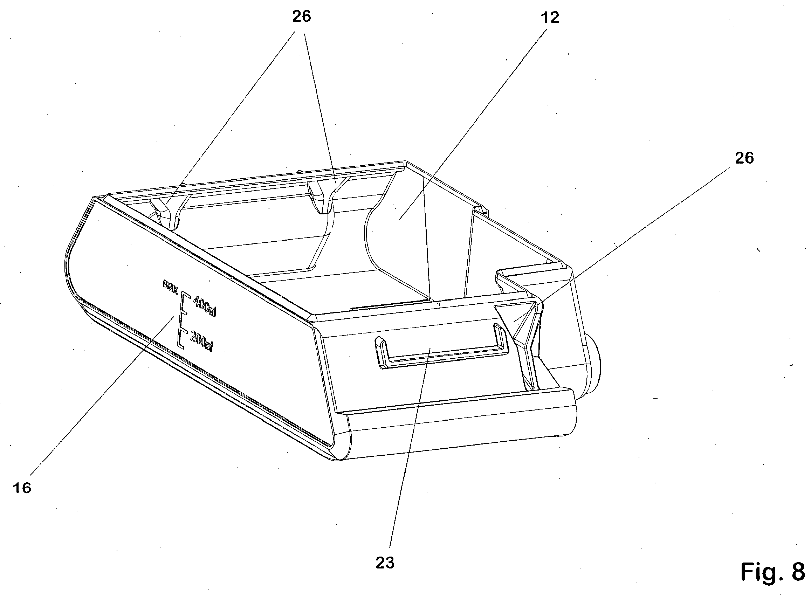

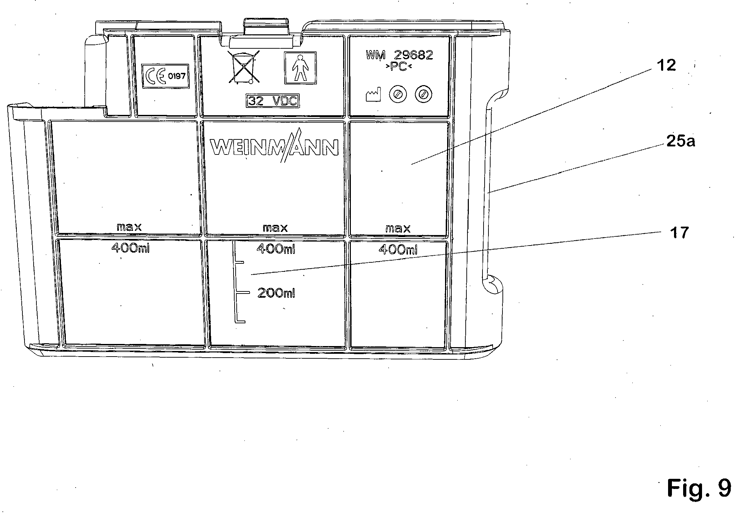

Das Gehäuseunterteil (12) (in den Figuren 8 und 9 dargestellt) weist eine Öffnung für das Heizelement (13) auf, das seit-lich in die Öffnung des Gehäuseunterteiles (12) eingeschraubt und mit einem O-Ring abgedichtet wird. Beim Anbau des Anfeuchters (11) an das Beatmungsgerät (1) wird das Heizelement (13) mit der Elektronik des Beatmungsgerätes (1) gekoppelt. Der Anfeuchter wird über das Beatmungsgerät (1) gesteuert.

Der Innenraum des Gehäuseunterteiles (12) dient zur Aufnahme des Wassers, wobei der Wasserstand über zwei Füllstandanzeigen (16 und 17) abgelesen werden kann. Eine Füllstandsanzeige (16) befinden sich im Seitenbereich des Unterteils und dient der Ermittlung des Wasserfüllstandes im Betrieb. Die andere Füllstandsanzeige befindet sich im Bodenbereich des Unterteils und dient der Ermittlung des Wasserfüllstandes (bei einem um 90° gekippten Anfeuchter) bei Befüllung durch die Öffnung (18, 20). Das Anfeuchtermodul (11) kann auf verschiedene Arten mit Wasser befüllt werden. Zum Einen kann er im zerlegten Zustand, also ohne Mittel-und Oberteil, befüllt werden, dafür dient die seitliche Füllstandanzeige (16) im Gehäuseunterteil (12). Zum Anderen kann das Anfeuchtermodul aber auch im zusammengebauten Zustand befüllt werden. Hierzu wird das Anfeuchtermodul (11), wie in Figur 6 dargestellt, geschwenkt und kann über den Lufteinlass (18) oder den Luftauslass (20) befüllt werden. Dafür ist eine zweite Füllstandanzeige (17) im Boden des Gehäuseunterteiles (12) zur Überwachung des Wasserstandes vorgesehen (ebenfalls in Fig.9 dargestellt). Im Bereich der Luft-Einlassöffnung (18) bzw. - Auslassöffnung (20) befindet sich jeweils eine erhöhte Kante (19, 20) die verhindert, dass beim Befüllen des Anfeuchters (11) das Wasser danebenlaufen kann. Die Flächen (19a, 21a) benachbart zu den Öffnungen 18 und 20 sind leicht trichterförmig ausgeführt, um das Wasser in Richtung der Öffnung zu führen. Wird der Anfeuchter (11) wie in Fig. 6 dargestellt mit Wasser befüllt, dient ein Überlaufschutz dazu, dass nicht zu viel Wasser eingefüllt werden kann. Der Überlaufschutz wird durch die Krümmungen der Luftkanäle (29 und 31) des Mittelteiles (14) realisiert. Da aus dem Innenraum keine Luft entweichen kann, wird durch das eingefüllte Wasser die Luft im Innenraum komprimiert und das Wasser steigt in den Luftkanälen (29 und 31) bis zu den Öffnungen (28 und 32) an. Wird der Anfeuchter (11) zurückgeschwenkt, läuft das Wasser in den Innenraum. Die gekrümmt angeordneten Luftkanäle (29 und 31) verhindern zudem ein Zurück- bzw. Auslaufen der Flüssigkeit aus dem Anfeuchter wenn dieser transportiert wird.

Fig. 8 zeigt zusätzlich ein weiteres Detail des Gehäuseunterteiles (12). Es sind Zentrierhilfen (26, 27) in Form von Führungsaufnahmen (26) des Gehäuseunterteiles (15) vorgesehen, in die entsprechenden Führungsstege (27) des Gehäuseoberteiles (15) eingreifen und so einen falschen Zusammenbau der Bauteile verhindern. Die Führungsaufnahmen (26) weisen eine trichterförmige Öffnung, die in einen Schlitz übergeht, auf. Die trichterförmige Öffnung dient zum Einführen des Führungssteges (27) in die Führungsaufnahme (26). Zudem sind die Führungsstege bzw. -aufnahmen asymmetrisch an Gehäuseunterteil (12) und Oberteil (15) angeordnet, was dazu beiträgt, dass es zu keiner falschen Montage führen kann.

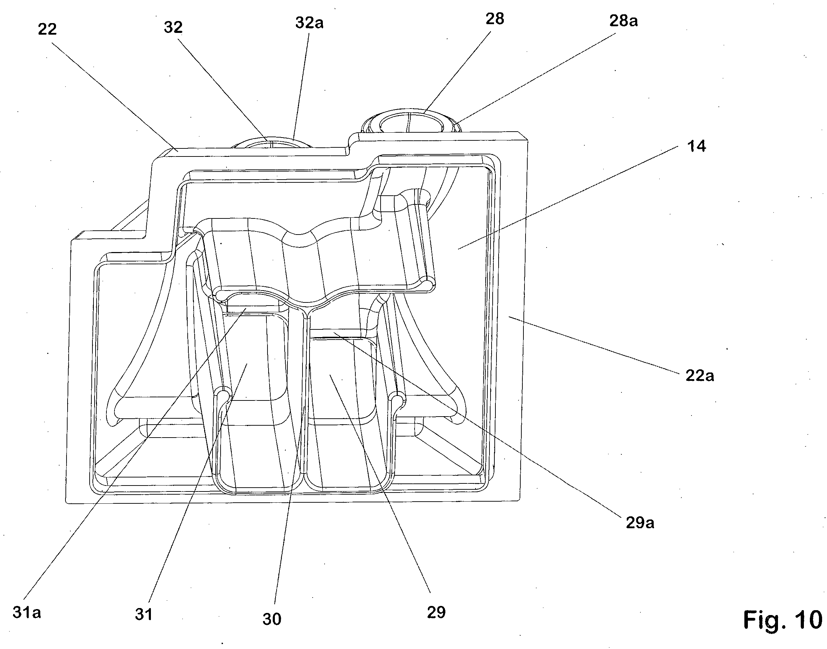

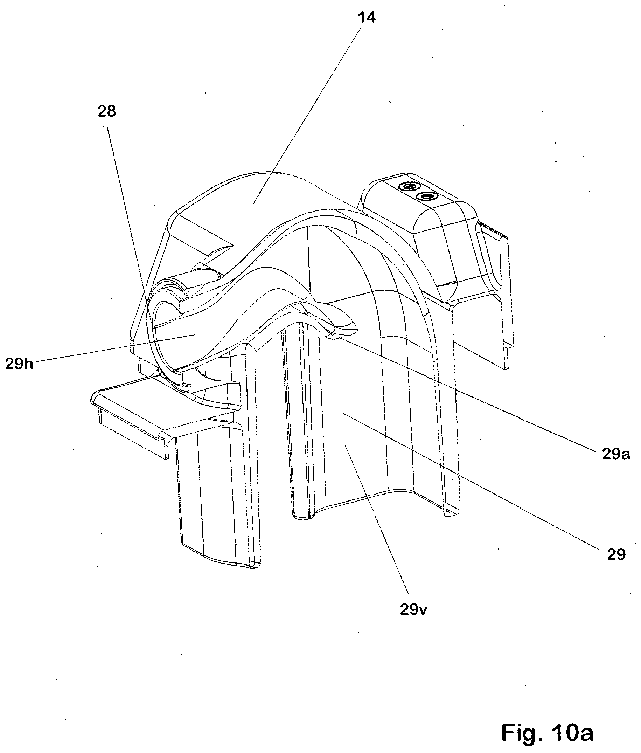

Fig. 10 zeigt das Mittelteil (14) des Atemluftanfeuchters (11). Es dient zum Einen der Luftführung durch den Anfeuchter und zum Anderen der Abdichtung. Durch das gewählte Material, in diesem Fall Silikon oder ein Elastomer welches im 2K-Verfahren hergestellt wurde, kann das Mittelteil (14) beide Anforderungen in Einem erfüllen. Das Mittelteil (14) ist zum Gehäuseoberteil (15) komplett geschlossen und zum Gehäuseunterteil mit dem Wasserreservoir (12) hin innen offen ausgeführt. Das Mittelteil (14) definiert dadurch einen genauen Luftweg durch den Anfeuchter. Der Luftstrom tritt durch die waagerechte Einlassöffnung (28) in einem Winkelbereich von 90° in das Mittelteil (14) ein, wird dann durch den Einlasskanal (29) derart geführt, das er zuerst in einem Winkel von ca. 110 - 120° nach oben und anschließend nach einem Bogen senkrecht nach unten in den Anfeuchterraum im Gehäuseunterteil (12) geleitet wird. Das Mittelteil leitet die Luft so senkrecht auf die Wasserfläche. Dort wird der Luftstrom angefeuchtet und durch den Luftkanal (31) in gleicher Weise zum Luftauslass (32) geführt. Durch das gewählte Material und den Konturverlauf der Kanäle durch das Mittelteil (14) wird der Luftstrom so beruhigt, dass keine zusätzliche Schalldämmung nötig ist. Der Dichtrand (22) des Mittelteiles (14) ist aus einem weicheren Elastomer bevorzugt Silikon ausgeführt und wird beispielsweise im 2K-Verfahren mit dem Mittelteil (14) verbunden.

Durch den Luftauslass (32) wird der Luftstrom über einen Anschlussstutzen (33) zurück in das Beatmungsgerät (1) geführt und dort durch den Beatmungsschlauch (5) dem Patienten über eine Beatmungsmaske oder -Brille zugeführt. Eine Trennwand (30) zwischen den Luftkanälen (29 und 31), die bis in das eingefüllte Wasser im Gehäuseunterteil (12) reicht, sorgt dafür, dass keine unangefeuchtete Luft in den Luftauslasskanal (31) gelangen kann. Der Dichtrand (28a, 32a) des Mittelteiles (14), welcher benachbart zum Einlass bzw. Auslass (28, 32) angeordnet ist und somit der Abdichtung zum Beatmungsgerät hin dient, ist aus einem weicheren Elastomer bevorzugt Silikon ausgeführt und wird beispielsweise im 2K-Verfahren mit dem Mittelteil (14) verbunden.

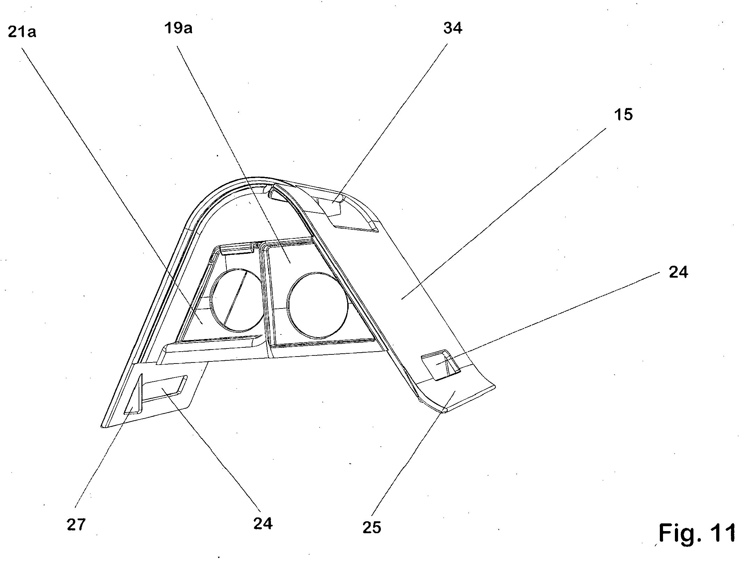

Fig.11 zeigt das Gehäuseoberteil (15) mit den Öffnungen (24), die beim Zusammenbau des Anfeuchters (11) über die Rasthaken (23) des Gehäuseunterteiles (12) greifen und den zusätzlichen Führungsstegen (27) zur Justierung des Gehäuseoberteiles (12). Das Oberteil weist zumindest einen Griff (25,34) auf. Eine Griffmulde (34) dient zum Tragen des Anfeuchters (11). Die Griffmulde (34) ist identisch zu einer Griffmulde am Beatmungsgerät (1) ausgeführt. Zum Lösen der Verbindung zwischen Ober- und Unterteil ist eine Grifffläche (25) im Bereich des Schenkels des Oberteiles (15) vorgesehen.

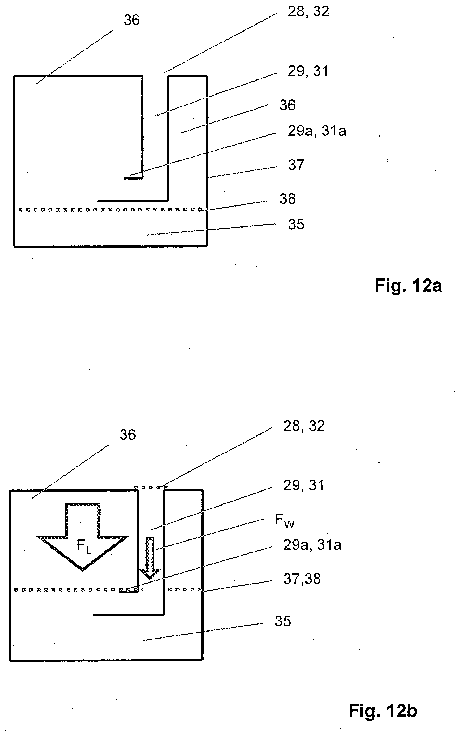

Figur 12 zeigt: Bei dem Befüllen des Anfeuchters (11) mit Wasser über die Öffnung (28 oder 32) steigt die Flüssigkeit im Wasseraufnahmeraum (35) des Anfeuchters langsam an. Das im Wasseraufnahmeraum (35) des Anfeuchters befindliche Volumen an Luft (36) entweicht zunächst sukzessiv durch die Öffnung (28,32). Steigt der Wasserstand (38) bis zum oberen Rand (29a, 31a) des Luftkanals (29,31) an, so kann die Luft nicht mehr entweichen, sie verbleibt im Luftraum (36). Das verbleibende Volumen an Luft wird mit weiter steigendem Wasserstand (38) im Wasseraufnahmeraum (35) des Anfeuchters komprimiert. Dies hat zur Folge, daß der Druck der eingeschlossenen Luft im Luftraum (36) des Anfeuchters auf das im Wasseraufnahmeraum (35) des Anfeuchters befindliche Wasser ansteigt. Ist der Druck (F_{L}) der eingeschlossenen Luft auf das im Wasseraufnahmeraum (35) des Anfeuchters befindliche Wasser, größer als die Kraft (F_{W}) des in dem Kanal (29,31) befindlichen eingefüllten Wassers, so ist die maximale Füllhöhe (37) des Anfeuchters (11) erreicht. Der Wasserstand (38) entspricht dann der maximalen Füllhöhe (37), die vom Anwender auch über die Füllstandanzeige (17) ablesbar ist. Bei weiterer Befüllung über die Öffnung (28, 32) fließt dann kein weiteres Wasser in den Wasseraufnahmeraum (35) der Anfeuchters. Das eingefüllte Wasser steigt dann in beiden Schenkeln des Luftkanals (29,31) bis zur Einfüllöffnung (28,32) empor, ohne die Wassermenge im Anfeuchter weiter zu erhöhen, und läuft über.

### Pos.-Nr.

- 1: Beatmungsgerät
- 2: Bedienfeld
- 3: Anzeige mit Touchscreen
- 4: Anschlusselement für Beatmungsschlauch
- 5: Beatmungsschlauch
- 6: Gehäuse Seitenwand
- 7: Integrierter Schalldämpfer in der Seitenwand (6)
- 8: Schnapphaken
- 9: Aufnahme für Schnapphaken
- 10: Entriegelung
- 11: Atemluftanfeuchter kpl.
- 12: Gehäuseunterteil Anfeuchter
- 13: Heizelement
- 14: Mittelteil
- 15: Gehäuseoberteil Anfeuchter
- 16: Füllstandanzeige im Seitenbereich des Unterteils
- 17: Füllstandanzeige im Bodenbereich des Unterteils
- 18: Lufteinlass
- 18a: Luftaustrittsöffnung aus Beatmungsgerät
- 19: Rand um Lufteinlass
- 19a: Fläche um Lufteinlass
- 20: Luftauslass
- 20a: Lufteintrittsöffnung in Beatmungsgerät
- 21: Rand um Luftauslass
- 21a: Fläche um Luftauslass
- 22: Dichtrand an (14)
- 23: Rasthaken in (12)
- 24: Öffnungen in (15)
- 25: Grifffläche am Gehäuseoberteil
- 25a: Aussparung für Griff am Gehäuseunterteil
- 26: Führungsaufnahme im Gehäuseunterteil (12)
- 27: Führungsstege am Gehäuseoberteil (15)
- 28: Lufteinlass im Mittelteil (14)
- 28a: Dichtrand am Einlass (28)
- 29: Luftkanal
- 30: Trennwand
- 31: Luftkanal zum Auslass im Mittelteil (14)
- 32: Luftauslass im Mittelteil (14)
- 32a: Dichtrand am Auslass (32)
- 33: Anschlussstutzen im Beatmungsgerät (1)
- 34: Griffmulde im Gehäuseoberteil (15)
- 35: Wasseraufnahmeraum
- 36: Luftraum
- 37: Füllhöhe
- 38: Wasserstand

## Patentansprüche

1. Atemluftanfeuchter zur Verwendung mit einem Beatmungsgerät bestehend aus einem Gehäuseunterteil (12) mit Heizelement (13), einem Gehäuseoberteil (15) und einem elastomeren Mittelteil (14) wobei das Mittelteil (14) eine Dichtauflage (22) zum Gehäuseunterteil (12) aufweist **dadurch gekennzeichnet, dass** der Anfeuchter (11) in einem Seitenbereich an das Beatmungsgerät (1) konnektiert wird und der Luftweg im Bypass durch den Anfeuchter (11) und wieder zurück in das Beatmungsgerät (1) geführt wird, wobei der Schlauchanschluss (4), an den ein Beatmungsschlauch mit Beatmungsmaske oder Beatmungsbrille angeschlossen werden kann, am Beatmungsgerät (1) verbleibt und wobei das Mittelteil (14) einstückig ausgebildet ist und Elemente zur Strömungsführung und/oder Strömungskanäle (28, 29, 30, 31, 32) und Dichtelemente (22, 28a, 32a) zur pneumatischen Abdichtung aufweist

2. Atemluftanfeuchter nach Anspruch 1 **dadurch gekennzeichnet, dass** das Beatmungsgerät im Seitenbereich eine Luftaustritts-(18a) und eine Lufteintrittsöffnung (20a) aufweist, die entweder im Bypass über den Schalldämpfer (6, 7) oder über den Anfeuchter (11) verbunden werden.

3. Atemluftanfeuchter nach zumindest einem der vorhergehenden Ansprüche **dadurch gekennzeichnet, dass** die Elemente zur Strömungsführung und/oder Strömungskanäle (28, 29, 30, 31, 32) mindestens eine Umlenkung der Luft von horizontal nach vertikal und/oder eine Umlenkung von vertikal in eine horizontale Strömungsrichtung zum Anfeuchterausgang hin bewirken.

4. Atemluftanfeuchter nach zumindest einem der vorhergehenden Ansprüche **dadurch gekennzeichnet, dass** die Elemente zur Strömungsführung und/oder Strömungskanäle (28, 29, 30, 31, 32) eine Aufteilung der Luftströmung in mindestens zwei Teilströme oberhalb der Wasseroberfläche bewirken und die Teilströme vor dem Luftauslass (20) wieder vereinigt werden.

5. Atemluftanfeuchter nach zumindest einem der vorhergehenden Ansprüche **dadurch gekennzeichnet, dass** das Mittelteil (14) aus mindestens 2 Elastomeren verschiedener Härtegrade (Shorehärten) besteht und die strömungsführenden Elemente des elastomeren Mittelteils (14) eine höhere Shorehärte als die Dicht(-auflage)-elemente (22, 28a, 32a) aufweisen.

6. Atemluftanfeuchter nach zumindest einem der vorhergehenden Ansprüche **dadurch gekennzeichnet, dass** das elastomere Mittelteil (14) rohrförmige Anschlussstellen bereitstellt die mit Öffnungen (18, 20) im Gehäuseoberteil gemeinsam jeweils einen Lufteinlass (18) und einen Luftauslass (20) bilden und zur luftführenden Kopplung mit dem Beatmungsgerät vorgesehen sind.

7. Atemluftanfeuchter nach zumindest einem der vorhergehenden Ansprüche **dadurch gekennzeichnet, dass** der Lufteinlass (18) und/oder der Luftauslass (20) auch als Einfüllöffnungen für das Wasser dienen.

8. Atemluftanfeuchter nach zumindest einem der vorhergehenden Ansprüche **dadurch gekennzeichnet, dass** Lufteinlass und Luftauslass benachbart angeordnet sind und eine Befüllung mit Wasser wahlweise durch eine der beiden Öffnungen vorgenommen werden kann.

9. Atemluftanfeuchter nach zumindest einem der vorhergehenden Ansprüche **dadurch gekennzeichnet, dass** das Mittelteil im Kopplungsbereich zum Beatmungsgerät Dichtelemente (28a, 32a) zur Abdichtung zum Beatmungsgerät hin aufweist.

10. Atemluftanfeuchter nach zumindest einem der vorhergehenden Ansprüche **dadurch gekennzeichnet, dass** Luftkanäle im Mittelteil beim Übergang zwischen vertikalen (29v, 31v) und horizontalen Abschnitten (29h, 31h) geometrische Überhöhungen (29a, 31a), in Form von oberen Rändern, aufweisen.

11. Atemluftanfeuchter nach zumindest einem der vorhergehenden Ansprüche **dadurch gekennzeichnet, dass** das Gehäuseoberteil (15) der mechanischen Abstützung des elastomeren Mittelteils, sowie, über Rastverbindungen (23, 24), der mechanischen Verriegelung mit dem Unterteil dient, wobei das Mittelteil zwischen Oberteil und Unterteil eingespannt wird und die Dicht(-auflage)elemente des elastomeren Mittelteils (14) den von Mittelteil (14) und Gehäuseunterteil (12) gemeinsam gebildeten Wasseraufnahmeraum (35) gegenüber der Umgebung abdichten.

12. Atemluftanfeuchter nach zumindest einem der vorhergehenden Ansprüche **dadurch gekennzeichnet, dass** im Gehäuseunterteil Führungsaufnahmen (26) zur Justierung des Gehäuseoberteiles (15) angeordnet sind, in die entsprechende Führungsstege (27) des Gehäuseoberteiles (15) greifen, wobei die Führungsstege und aufnahmen (26, 27) asymmetrisch an Gehäuseunterteil (12) und Oberteil 1 (15) angeordnet sind um eine definierte Orientierung bei der Montage vorzugeben.

13. Atemluftanfeuchter nach Anspruch 12 **dadurch gekennzeichnet, dass** die Führungsaufnahmen (26) jeweils eine trichterförmige Öffnung aufweisen die zum Einführen der Führungsstege (27) in die Führungsaufnahmen (26) dienen.

14. Atemluftanfeuchter nach zumindest einem der vorhergehenden Ansprüche **dadurch gekennzeichnet, dass** im Gehäuseunterteil zwei von außen sichtbare Füllstandanzeigen (16, 17) angeordnet sind.

15. Atemluftanfeuchter nach zumindest einem der vorhergehenden Ansprüche **dadurch gekennzeichnet, dass** der Atemluftanfeuchter lediglich aus vier Bauteilen, nämlich einem Gehäuseunterteil (12) mit Heizelement (13), einem Gehäuseoberteil (15) und einem elastomeren Mittelteil besteht.

16. Atemluftanfeuchter nach zumindest einem der vorhergehenden Ansprüche **dadurch gekennzeichnet, dass** das Mitteilteil Elemente zur Strömungsführung und/oder Strömungskanäle (28, 29, 30, 31, 32) aufweist, die aufgrund ihrer räumlichen Gestalt bei einem werkzeugbasierten Herstellungsprozesses eine elastische Nachgiebigkeit des Mittelteiles zu deren Entformung voraussetzen.

## Claims

1. A respiratory humidifier for use with a ventilator, consisting of a lower part of housing (12) with a heating element (13), a top part of housing (15) and an elastomeric middle part (14), with the middle part (14) having a sealing support (22) to the lower part of housing (12) **characterized by** the fact that the humidifier (11) is connected on the side area to the ventilator (1) and that the airway runs through the bypass to the humidifier (11) and back again into the ventilator (1), with the tube connection (4), to which a ventilation tube with ventilation mask or nasal cannula can be connected, staying on the ventilator (1) and with the middle part (14) being designed as one piece and which has elements for flow guidance and/or flow channels (28, 29, 30, 31, 32) and sealing elements (22, 28a, 32a) for pneumatic sealing.

2. Respiratory humidifier, characterized according to claim 1, by the fact that the ventilator has, in the side area, an air outlet (18a) and an air inlet opening (20a), which are either connected in the bypass via the baffle (sound insulation)(6, 7) or via the humidifier (11).

3. Respiratory humidifier, characterized according to at least one of the previous claims, by the fact that the elements for the flow guidance and/or flow channels (28, 29, 30, 31, 32) cause at least one redirection of the air from horizontal to vertical and/or a redirection from a vertical to a horizontal direction of flow to the humidifier outlet.

4. Respiratory humidifier, characterized according to at least one of the previous claims, by the fact that the elements for the flow guidance and/or flow channels (28, 29, 30, 31, 32) cause a division of the flow of air into at least two partial streams above the water surface, with the partial streams being reunited upstream of the air outlet (20).

5. Respiratory humidifier, characterized according to at least one of the previous claims, by the fact that the middle part (14) consists of at least two elastomers of different degrees of hardness (Shore hardness) and that the flow-carrying elements of the elastomeric middle part (14) have a higher Shore hardness than the sealing (support) elements (22, 28a, 32a).

6. Respiratory humidifier, characterized according to at least one of the previous claims, by the fact that the elastomeric middle part (14) provides tubular connection terminals which, with openings (18, 20) in the top part of housing, together form an air inlet (18) and an air outlet (20) and which have an air-conducting coupling with the ventilator.

7. Respiratory humidifier, characterized according to at least one of the previous claims, by the fact that the air inlet (18) and/or the air outlet (20) also serve as filler necks for the water.

8. Respiratory humidifier, characterized according to at least one of the previous claims, by the fact that the air inlet and air outlet are arranged adjacent to one another and filling with water can optionally be made through one of the two openings.

9. Respiratory humidifier, characterized according to at least one of the previous claims, by the fact that the middle part, in the coupling area to the ventilator, has sealing elements (28a, 32a) to seal it to the ventilator.

10. Respiratory humidifier, characterized according to at least one of the previous claims, by the fact that the air channels in the middle part, during the transition between vertical (29v, 31v) and horizontal sections (29h, 31h), have geometric elevations (29a, 31a), in the form of upper edges.

11. Respiratory humidifier, characterized according to at least one of the previous claims, by the fact that the top part of housing (15) serves as mechanical support for the elastomeric middle part and as mechanical locking with the lower part by means of snap connections (23, 24), with the middle part being clamped between the upper part and lower part and the sealing (support) elements of the elastomeric middle part (14) sealing the water receiving space (35), which is formed by both the middle part (14) and the bottom part of housing (12), from the environment.

12. Respiratory humidifier, characterized according to at least one of the previous claims, by the fact that guide receptacles (26) for adjusting the top part of housing (15) are arranged in the lower part of housing, which are gripped by the corresponding guide rings (27) of the top part of housing (15), with the guide rings and guide receptacles (26, 27) being arranged asymmetrically on the lower part of housing (12) and the top part (15) in order to provide a defined orientation during assembly.

13. Respiratory humidifier, characterized according to claim 12, by the fact that the guide receptacles (26) each have a funnel-shaped opening for receiving the guide rings (27) in the guide receptacles (26).

14. Respiratory humidifier, characterized according to at least one of the previous claims, by the fact that there are two level indicators (16, 17), visible from the outside, arranged in the lower part of housing.

15. Respiratory humidifier, characterized according to at least one of the previous claims, by the fact that the respiratory humidifier consists of only four components, namely a lower part of housing (12) with a heating element (13), a top part of housing (15) and a elastomeric middle part.

16. Respiratory humidifier, characterized according to at least one of the previous claims, by the fact that the middle part has elements for the flow guidance and/or flow channels (28, 29, 30, 31, 32) which, owing to their spatial shape in the case of a tool-based manufacturing process, give the middle part an elastic resilience during the demolding process.

## Revendications

1. Humidificateur pour utilisation avec un respirateur se composant d'une embase (12) avec élément chauffant (13), d'une partie supérieure (15) et d'une partie centrale en élastomère (14), la partie centrale (14) présentant une surface d'appui étanche (22) sur l'embase de l'appareil(12), **caractérisé en ce que** l'humidificateur est connecté dans une partie latérale du respirateur (1) et **en ce que** le flux d'air est conduit et retourné dans la dérivation par l'humidificateur (11) dans le respirateur (1), le raccord de tuyau (4) auquel un masque ou une lunette respiratoire peuvent être raccordés restant fixé sur le respirateur (1) et la partie centrale (14) étant formée d'une seule pièce et présentant des éléments d'écoulement et/ou canaux d'écoulement (28, 29, 30, 31, 32) et des éléments d'étanchéité (22, 28a, 32a) destinés à l'étanchéité pneumatique.

2. Humidificateur selon la revendication 1 **caractérisé en ce que** le respirateur présente sur le côté une ouverture de sortie d'air (18a) et une ouverture d'entrée d'air (20a), lesquelles sont reliées à la dérivation soit via le silencieux (6, 7), soit via l'humidificateur (11).

3. Humidificateur selon au moins l'une des revendications précédentes **caractérisé en ce que** les éléments d'écoulement et/ou canaux d'écoulement (28, 29, 30, 31, 32) engendrent au moins une dérivation de l'air du sens d'écoulement horizontal dans le sens d'écoulement vertical et/ou une dérivation du sens d'écoulement vertical dans un sens d'écoulement horizontal par rapport à la sortie de l'humidificateur.

4. Humidificateur selon au moins l'une des revendications précédentes **caractérisé en ce que** les éléments d'écoulement et/ou canaux d'écoulement (28, 29, 30, 31, 32) entraînent une répartition du flux d'air en au moins deux flux partiels au-dessus de la surface de l'eau et **en ce que** les flux partiels sont réunis avant la sortie d'air (20).

5. Humidificateur selon au moins l'une des revendications précédentes **caractérisé en ce que** la partie centrale (14) se compose d'au moins 2 élastomères de dureté différente (duretés Shore) et **en ce que** les éléments d'écoulement de la partie centrale en élastomères (14) présentent une dureté Shore supérieure à celle des éléments (d'appui) étanches (22, 28a, 32a).

6. Humidificateur selon au moins l'une des revendications précédentes **caractérisé en ce que** la partie centrale en élastomères (14) met à disposition des points de raccordement en forme de tuyau, lesquels forment ensemble avec les ouvertures (18, 20) dans la partie supérieure une entrée d'air (18) et une sortie d'air (20) pour servir de raccord de circulation d'air avec le respirateur.

7. Humidificateur selon au moins l'une des revendications précédentes **caractérisé en ce que** l'entrée d'air (18) et/ou la sortie d'air (20) servent également d'orifices de remplissage d'eau.

8. Humidificateur selon au moins l'une des revendications précédentes **caractérisé en ce que** l'entrée d'air et la sortie d'air sont juxtaposées et **en ce qu'**elles peuvent être employés en guise d'orifices de remplissage d'eau.

9. Humidificateur selon au moins l'une des revendications précédentes **caractérisé en ce que** la partie centrale présente dans la zone de liaison avec le respirateur des éléments d'étanchéité (28a, 32a) destinés à assurer l'étanchéité du respirateur.

10. Humidificateur selon au moins l'une des revendications précédentes **caractérisé en ce que** les canaux d'air de la partie centrale présentent, lors du passage entre les sections verticales (29v, 31v) et les sections horizontales (29h, 31h), des surépaisseurs géométriques (29a, 31a) formant des bords supérieurs.

11. Humidificateur selon au moins l'une des revendications précédentes **caractérisé en ce que** la partie supérieure de l'appareil (15) sert d'appui mécanique à la partie centrale en élastomères ainsi qu'aux liaisons par encliquetage (23, 24), la partie centrale étant enserrée entre l'embase et la partie supérieure et les éléments (d'appui) d'étanchéité de la partie centrale en élastomères (14) assurant l'étanchéité par rapport à l'environnement du compartiment d'eau (35) formé par la partie centrale (14) et l'embase de l'appareil (12).

12. Humidificateur selon au moins l'une des revendications précédentes **caractérisé en ce que** des logements de guidage (26) destinés à l'ajustement de la partie supérieure de l'appareil (15) sont prévus dans l'embase de l'appareil et dans lesquels s'emboîtent des ergots de guidage (27) de la partie supérieure de l'appareil (15), les ergots et logements de guidage (26, 27) étant positionnés de manière asymétrique sur l'embase (12) et la partie supérieure de l'appareil (15) afin de fournir une orientation définie lors du montage.

13. Humidificateur selon la revendication 12 **caractérisé en ce que** les logements de guidage (26) présentent une ouverture en forme d'entonnoir destinée à amener les ergots de guidage (27) dans les logements de guidage (26).

14. Humidificateur selon au moins l'une des revendications précédentes **caractérisé en ce que** deux indicateurs de niveaux visibles depuis l'extérieur (16, 17) équipent l'embase de l'appareil.

15. Humidificateur selon au moins l'une des revendications précédentes **caractérisé en ce que** l'humidificateur se compose seulement de quatre éléments, à savoir une embase (12) avec élément chauffant (13), une partie supérieure (15) et une partie centrale en élastomères.

16. Humidificateur selon au moins l'une des revendications précédentes **caractérisé en ce que** la partie centrale présente des éléments d'écoulement et/ou des canaux d'écoulement (28, 29, 30, 31, 32), lesquels, en raison de leur conception spatiale dans le cadre d'un processus de fabrication employant des outils, impliquent une souplesse élastique de la partie centrale afin de permettre leur déformation.
